(19) Europäisches Patentamt
European Patent Office
Office européen des brevets

(11) Publication number: **0 000 559**
**B1**

(12) # EUROPEAN PATENT SPECIFICATION

(45) Date of publication of patent specification: **05.10.83**

(51) Int. Cl.$^3$: **C 07 C 103/52,**
A 61 K 37/02

(21) Application number: **78100463.5**

(22) Date of filing: **21.07.78**

(54) **Pentapeptide-N-alkylamides and their acid addition salts, methods for preparation of these compounds and pharmaceutical formulations containing them.**

(30) Priority: **22.07.77 GB 3090977**
**24.11.77 GB 4898077**

(43) Date of publication of application:
**07.02.79 Bulletin 79/3**

(45) Publication of the grant of the patent:
**05.10.83 Bulletin 83/40**

(84) Designated Contracting States:
**CH DE FR NL SE**

(56) References cited:
**FR - A - 2 338 925**
**FR - A - 2 347 336**
**FR - A - 2 359 817**
**FR - A - 2 364 890**
**FR - A - 2 365 553**

**UNLISTED DRUGS (Edit. Special Libr. associat.) Vol. 29, page 190 n (1977)**

**UNLISTED DRUGS — April 1978 — ICI 122984**

(73) Proprietor: **THE WELLCOME FOUNDATION LIMITED**
**183-193 Euston Road**
**London NW1 2BP (GB)**

(72) Inventor: **Wilkinson, Samuel**
**12 Bevington Road**
**Beckenham, Kent (GB)**

(74) Representative: **Berg, Wilhelm, Dr. et al,**
**Dr. Berg, Dipl.-Ing. Stapf, Dipl.-Ing. Schwabe, Dr. Dr. Sandmair Patentanwälte Postfach 860245**
**D-8000 München 86 (DE)**

(56) References cited:
**UNLISTED DRUGS March 1978 — ICI 118736**

**UNLISTED DRUGS March 1978 — ICI 116750**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

**EP 0 000 559 B1**

# Pentapeptide-N-alkylamides and their acid addition salts, methods for preparation of these compounds and pharmaceutical formulations containing them

This invention relates to pentapeptide-N-alkylamides and their acid addition salts; to the preparation of such compounds; to formulations containing such compounds and the preparation of such formulations; and to the use of the compounds in human and veterinary medicine.

The present invention more particularly relates to the peptides of the formula (I):

H.Tyr.*D*-Met.Gly.$X^4$.$X^5$.NHR (I)

and their acid addition salts.

In formula (I),

$X^4$ is *L*-phenylalanyl or *L*-4-nitrophenylalanyl;

$X^5$ is *D*-leucyl, *D*-methionyl, *D*-prolyl or *L*-prolyl; and

R is an alkyl group of 1 to 5 carbon atoms;

*provided that*

when $X^4$ is *L*-phenylalanyl

*then* $X^5$ is *D*-prolyl.

The proviso is necessary in order to exclude a compound taught as having been investigated for opiate activity in Bajusz et al., *Acta Biochim. et Biophys. Acad. Sci. Hung.,* Vol. 11 (4), pages 305—309 (1976).

In formula (I), the alkyl group R may in particular have for example 1 or 2 carbon atoms.

As preferred subclasses within formula (I) may be mentioned those peptides wherein $X^4$ is *L*-4-nitrophenylalanyl and wherein $X^5$ is *L*-prolyl.

In the French patents 2 347 336 and 2 338 925 derivatives of pentapeptides are also disclosed which are, however, different from the pentapeptide-N-alkylamides as claimed by the present invention.

The abbreviations used herein for amino acids and their radicals are those conventional in the art and may be found in, for example, *Biochemistry, 11*, 1726 (1972). In the above and throughout the following all references are to the *L*-amino acids and their radicals except in the case of glycine and unless otherwise stated.

The peptides of formula (I) and their acid addition salts, when assessed by a number of standard pharmacological procedures, have been found both to induce and to maintain anaesthesia in laboratory animals including rats and mice. The compounds are effective in this respect when administered by a variety of routes including parenteral, for example by intravenous or intracerebroventricular injection. Illustrative of the anaesthetic effects of the compounds are the following, which should be understood to be non-limiting.

(i) *Abolition of the righting reflex.* This is characteristic of recognised anaesthetic agents such as chloral hydrate (2,2,2-trichloro-1,1-ethandiol), urethan (ethyl carbamate) and the barbiturates (derivatives of barbituric acid). An animal lacking this reflex does not roll over or attempt to regain its normal posture when paced on its back.

(ii) *Abolition of the pinnal reflex.* In this procedure a wire or similar probe is introduced into the ear pinna; in the normal (control) animal there is a resultant reflex twitch or shake of the affected pinna.

(iii) *Abolition of the corneal reflex.* In this procedure the cornea is lightly touched with a wire or similar; in the normal (control) animal there is a resultant reflex blink of the eyelids. This reflex is of clinical importance in man in that it is one of the last reflexes to be abolished during the induction of general anaesthesia.

Each of the foregoing effects (i), (ii) and (iii) may be reversed by administration of the known narcotic antagonist naloxone (1-N-allyl-7,8-dihydro-14-hydroxynormorphinone). However it has been found that morphine itself does *not* abolish the righting reflex in laboratory animals such as mice when administered by acute bolus injection in up to lethal doses.

In the acid addition salts of the peptides of formula (I) the activity resides in the base and the acid is of less importance although for therapeutic purposes it is preferably pharmacologically and pharmaceutically acceptable to the recipient. Examples of such suitable acids include

(a) *mineral acids:* hydrochloric, hydrobromic, phosphoric, metaphosphoric, nitric and sulphuric acids;

(b) *organic acids*: tartaric, acetic, citric, malic, lactic, fumaric, benzoic, glycollic, gluconic, gulonic, succinic and arylsulphonic, for example *p*-toluenesulphonic, acids. The pharmaceutically and pharmacologically acceptable acid addition salts together with those salts which are not so acceptable (for example salts of hydrofluoric and perchloric acids) have utility in isolation and purification of the bases, and of course the unacceptable salts are also valuable in the preparation of the acceptable salts by techniques well known in the art.

The peptides of formula (I) and their pharmacologically and pharmaceutically acceptable acid addition salts may be used in the fields of both human and veterinary medicine for the induction and/or maintenance of anaesthesia in a mammal.

A peptide or an acid addition salt thereof may be administered either alone as the sole anaesthetic agent or in combination with one or more other substances which may complement and/or supplement its activity. Such additional substances may be administered before, simultaneously with or after administration of the peptide or acid addition salt thereof and in the case of simultaneous administration the various agents may be administered either as separate doses or as a combination formulation.

As one possibilty the peptide or acid addition salt thereof may be administered subsequent to administration of a benzodiazepine tranquillizer such as chlordiazepoxide (7-chloro-2-methylamino-5-phenyl-3H-1,4-benzodiazepine-4-oxide), diazepam (7-chloro-1,3-dihydro-1-methyl-5-phenyl-2H-1,4-benzodiazepin-2-one) and oxazepam (7-chloro-1,3-dihydro-3-hydroxy-5-phenyl-2H-1,4-benzodiazepin-2-one).

As another possibility the peptide or acid addition salt thereof may be administered for the maintenance of anaesthesia after this has been initially induced by the previous administration of another anaesthetic agent, for example a barbiturate such as thiopental sodium (sodium 5-ethyl-5-(1-methylbutyl)-2-thiobarbiturate).

A particular utility for the peptides of formula (I) and their pharmacologically and pharmaceutically acceptable acid addition salts, within the field of anaesthesia, is the induction and/or maintenance of the state referred to as "neuroleptanalgesia", a condition characterised by quiescence, psychic indifference to environmental stimuli, and analgesia (see, for example, *Dorland's Illustrated Medical Dictionary*, twenty-fifth edition, published by W.B. Saunders, 1974, at page 1041, and *"The Pharmacological Basis of Therapeutics"*, Goodman, L.S. and Gilman, A. eds., fifth edition, published by MacMillan Publishing Co. Inc., 1975, especially at Chapter 8, pages 97 to 101). This condition is recognised by clinicians as desirable for enabling the performance of procedures such as bronchoscopy, X-ray studies, burn dressings and cystoscopy wherein a degree of patient cooperation is of value, and a fixed-dose combination comprising the narcotic analgesic fentanyl citrate (N-(1-phenethyl-4-piperidyl)propionanilide citrate) and the neuroleptic agent droperidol (1-{1-[3-(*p*-fluorobenzoyl)propyl]-1,2,3,6-tetrahydro-4-pyridyl}-2-benzimidazolinone) has found acceptance for use in such circumstances.

Heretofore neuroleptanalgesia has been achievable only upon administration of such a narcotic analgesic plus neuroleptic drug combination as that above mentioned. The peptides of formula (I) and their acceptable acid addition salts are thus an important clinical advance and valuable addition to the armamentarium of the medical and veterinary professions in alone enabling this result, without any additional medication being required.

In addition to their ability both to induce and maintain anaesthesia, as hereinbefore described, the peptides of formula (I) and their acid addition salts have been found to exhibit morphie agonist activity. As generally accepted and as the term is used herein, a morphine agonist is a compound the biological activity of which mimics that of the natural alkaloid.

The pharmacological properties and therapeutic uses of morphine are well documented in the literature, see for example *"The Pharmacological Basis of Therapeutics*, Goodman, L.S. and Gilman, A. eds., published by The MacMillan Company, New York, third edition (1965) especially at Chapter 15, pages 247 to 266, and *"Martindale: The Extra Pharmacopoeia*, Blacow, N.W. ed., published by The Pharmaceutical Press, London, twenty-sixth edition (1972) especially at pages 1100 to 1106. As is well known however (Goodman, L.S. et al., *loc. cit.*, Chapter 16) repeated adminstration of morphine can lead to the recipient developing an addiction to the drug and tolerance to its effects and to his manifesting withdrawal symptoms when administration is discontinued. For many years therefore research has been conducted with the aim of obtaining a compound having the activity spectrum of morphine while lacking its disadvantages.

The morphine agonist properties of the peptides of formula (I) and their derivatives as hereinbefore defined include the following, which are given solely by way of illustration and should be understood to be non-limiting.

(A) *In vitro:*

(i) Inhibition of neurally evoked contractions of the isolated mouse vas deferens when tested by the method of Hughes et al (*Brain Research, 88* (1975) 296) (using pulses at 0.1 Hz), the inhibition being abolished by the known narcotic antagonist naloxone (1-N-allyl-7,8-dihydro-14-hydroxy normorphinone).

(ii) Reduction of electrically-induced contractions of the isolated guinea-pig ileum when prepared for stimulation after the manner of Paton (*Briton. J. Pharmacol., 12* (1957) 119—127). (Each intestinal segment was impaled by the anode and suspended with a 2—3 g load. Stimulus parameters: frequency: 0.1 Hz; duration: 0.4 ms; voltage (supramaximal) 30—40 V; the contractions were transduced isotonically).

(B) *In vivo:*

(i) The compounds exhibit analgesic activity, for example they are effective in mice in the "hot plate" procedure standard in the art when tested by a modification of the method of Eddy, N.B. et al. (*J. Pharm. Exp. Therap. 107,* 385 (1953)), the compounds being administered by intracerebroventricular injection, and this activity is abolished by naloxone. As a further illustration of analgesic activity the peptides of formula (I) are effective in mice in the abolition of acetic-acid induced writhing when screened by a modification of the method of Hendershot L.C. and Forsaith J., *J. of Pharm. and Exp. Ther.,* 1959, Vol. 125, page 237; the compounds being administered orally; and the abolition being reversed by naloxone.

(ii) The compounds exhibit antitussive activity, for example when tested in guinea-pigs according to the method of Boura et al, *Brit. J. Pharmacol, 39* (1970) 225.

(iii) The compounds exhibit antidiarrhoeal activity, for example they are effective in reducing castor oil-induced diarrhoea in rats.

Because of their morphine agonist activity already alluded to the peptides of formula (I) together with their pharmacologically and pharmaceutically acceptable acid addition salts may be used in the treatment of mammals in the fields of both human and veterinary medicine in any condition where an agent with a morphine-like effect is indicated. Specific utilities that may be mentioned, by way of example, include the following:

(1) The relief of pain (analgesic), for example pain arising from spasm of smooth muscle as in renal or biliary colic, pain due to terminal illness such as cancer, pain in the post-operative period, and obstetrical pain.

(2) Sedation, for example in pre-anaesthetic medication; tranquillization; the induction of sleep, especially where sleeplessness is due to pain or cough; and the relief of anxiety in general.

(3) The suppression of cough.

(4) The relief of dyspnoea, for example that of acute left ventricular failure or pulmonary oedema.

(5) The induction of constipation, for example after ileostomy or colostomy, and the treatment of diarrhoea and dysentry.

(6) The induction of euphoria and the treatment of depression, for example when allied to the relief of pain in terminal illness such as cancer.

For each of the utilities recited hereinbefore for the peptides of formula (I) and their acid addition salts, that is to say, whether for use for the induction and/or maintenance of anaesthesia (for example the induction and/or maintenance of neuroleptanalgesia) or for use in a condition where an agent with a morphine-like effect is indicated (for example the utilities specifically identified hereinbefore under (1), (2), (3), (4), (5) or (6)) the amount required of the peptide or acid addition salt thereof (hereafter referred to as the active ingredient) will vary with the route of administration and with the nature and requried extent of the desired effect, and will ultimately be at the discretion of the physician or veterinarian. In general however for each of these utilities the dosage will be in the range 0.0025 $\mu$g to 40 mg per kilogram bodyweight of mammal, preferably 0.01 $\mu$g to 4.0 mg/kg and optimally 0.25 to 400 $\mu$g/kg (all dosages calculated with reference to the peptide base).

The active ingredients may be administered by any route appropriate to the effect to be achieved, suitable routes including oral, rectal, nasal, topical (buccal), vaginal and parenteral (including subcutaneous, intramuscular and intravenous). It will be appreciated that the preferred route will vary with the effect to be achieved and thus for example in the relief of obstetrical pain administration directly into the spinal cord may be advantageous.

While it is possible for the active ingredients to be administered as the raw chemical it is preferable to present them as pharmaceutical formulation preparation.

The formulations, both veterinary and for human use, of the present invention comprise an active ingredient, as above defined, together with one or more acceptable carriers therefor and optionally

other therapeutic ingredients. The carrier(s) must be "acceptable" in the sense of being compatible with the other ingredients of the formulation and not deleterious to the recipient thereof. Desirably the formulations should not include oxidising agents and other substances with which peptides are known to be incompatible.

The formulations include those suitable for oral, rectal, nasal, topical (buccal), vaginal or parenteral (including subcutaneous, intramuscular and intravenous) administration, although the most suitable route in any given case will depend upon for example the active ingredient and the condition to be treated. The formulations may conveniently be presented in unit dosage form and may be prepared by any of the methods well known in the art of pharmacy. All methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product into the desired formulation.

Formulations of the present invention suitable for oral administration may be presented as discrete units such as capsules, cachets or tablets each containing a predetermined amount of the active ingredient; as a powder or granules; or as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tablets may be prepared by compressing in a suitable machine, the active ingredient in a free-flowing form such as a powder or granules, optionally mixed with a binder, lubricant, inert diluent, lubricating, surface active or dispersing agent. Moulded tablets may be made by moulding in a suitable machine, a mixture of the powdered compound moistened with an inert liquid diluent.

Orally ingestible formulations comprising the compounds of formula (I) may be rendered less liable to any abuse, for example, unauthorised intravenous administration, by including in the formulation an appropriate amount of a specific opiate antagonist such as naloxone (1-*N*-allyl-7,8-dihydro-14-hydroxy normorphinone) which is ineffective in man upon oral adminstration but which is effective upon administration by the intravenous route. The medicinal effect of the compounds of formula (I) would thus be unaffected when such a formulation was taken orally as intended but would be antagonized and countered by the naloxone if intravenous administration were effected.

Formulations for rectal administration may be presented as a suppository with the usual carriers such as cocoa butter, while a suitable formulation for nasal administration is nasal drops comprising the active ingredient in aqueous or oily solution.

Formulations suitable for topical administration in the mouth include lozenges comprising the active ingredient in a flavoured basis, usually sucreose and acacia or tragacanth; and pastilles comprising the active ingredient in an inert basis such as gelatin and glycerin, or sucrose and acacia.

Formulations suitable for vaginal administration may be presented as pessaries, creams, pastes or spray formulations containing in addition to the active ingredient such carriers as are known in the art to be appropriate.

Formulations suitable for parenteral administration conveniently comprise sterile aqueous solutions of the active ingredient, which solutions are preferably isotonic with the blood of the recipient. Such formulations may be conveniently prepared by dissolving the solid active ingredient in water to produce an aqueous solution, and rendering said solution sterile and isotonic with the blood of the recipient. The formulations may be presented in unit — or in multi-dose containers, for example sealed ampoules or vials.

Formulations suitable for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose.

It should be understood that in addition to the aforementioned ingredients the formulations of this invention may include one or more additional ingredients such as diluents, buffers, flavouring agents, binders, surface active agents, thickeners, lubricants, preservatives (including anti-oxidants) and the like.

Where the formulation, for human or for veterinary use, is presented in unit dosage form, for example those unit dosage forms specifically mentioned above, each unit thereof conveniently contains the active ingredient (as above defined) in an amount in the range 0.125 $\mu$g to 2 g, preferably 1.25 $\mu$g to 200 mg and optimally 12.5 $\mu$g to 20 mg (all weights calculated with reference to the peptide base).

The peptides of formula (I) and their acid addition salts may be prepared by any of the methods known in the art for the preparation of compounds of analogous structure. Thus they may be formed by the sequential coupling of appropriate amino acids using either classical methods of peptide synthesis or solid phase procedures, or by the initial preparation and subsequent coupling of peptide sub-units.

Such reactions may be effected by, for example, activating the carboxlyic acid group of the ingoing amino acid and protecting the non-reacting amino and carboxylic acid groups. Such techniques

are standard in the peptide art. Details of suitable activating and protecting (masking) groups and of suitable reaction conditions (both for the coupling reactions and for the removal of protecting groups) giving the minimum of racemisation may be found in the following literature, which is given purely by way of exemplification and which is intended to be neither exhaustive nor limiting.

Schröder and Lübke, *"The Peptides"* (Academic Press) (1965).
Bellean and Malek, *J. Am. Chem. Soc., 90,* 165 (1968).
Tilak, *Tetrahedron Letters,* 849 (1970).
Beyerman, *Helv. Chim. Acta., 56,* 1729 (1973).
Steward and Young, *"Solid Phase Peptide Synthesis"* (W.H. Freeman and Co.) (1969).

Depending upon the reaction conditions the peptides of formula (I) are obtained in the form of the free base or as an acid addition salt thereof. The acid addition salts may be converted into the free bases or salts of other acids, and the bases may be converted into acid addition salts thereof, by techniques well known in the art.

The peptides of formula (I) and acid addition salts thereof may thus be prepared by condensing a reagent (II)

$$H—Y^1—OH \qquad (II)$$

wherein $Y^1$ is selected from the radical —Tyr— and a partial radical sequence having the radical —Tyr— at its N-terminal end and from thereon corresponding to formula (I), with a reagent (III)

$$H—Y^2 \qquad (III)$$

wherein $Y^2$ corresponds to the balance of the above defined product, the reagents (II) and (III) being optionally protected and/or activated where and as appropriate; followed if necessary and as appropriate by one or both of the steps of deprotection of the product and conversion of the product into the base or an acid addition salt thereof.

It will be appreciated that the peptides of formula (I) may also be prepared by reaction of a corresponding peptide alkyl ester, for example the methyl ester, with an appropriate monoalkylamine.

The following Examples serve to illustrate the present invention but should not be construed as in any way providing a limitation thereof. All temperatures are in degrees Celsius.

## Experimental Section

The following abbreviations are used throughout

| | |
|---|---|
| HOBT | 1-hydroxybenzotriazole |
| DCCI | dicyclohexylcarbodiimide |
| DMF | dimethylformamide |
| BOC | tertiary butyloxycarbonyl |
| HOAc | acetic acid |
| MeOH | methanol |

Peptides were examined by tlc on Merck silicagel plates with the following solvent systems:

1. n-Butanol:acetic acid:water (3:1:1)
2. Chloroform:methanol:32% acetic acid (120:90:40)
3. Chloroform:methanol:32% acetic acid (120:90:5)
4. Chloroform:methanol:32% aq. ammonia (120:90:5)

All amino acids were of the *L*-configuration unless otherwise stated.

Optical rotations were determined on a Bendix® NPL automatic polarimeter.

The amino acid compositions of peptide hydrolysates (6*N*.HCl at 110° for 24 hours in evacuated sealed tubes) were determined with a Beckman-Spinco® Model 120C amino acid analyser or with a Rank Chromostak® amino acid analyser.

The following general procedures were used throughout the syntheses of the peptides.

(a) Couplings were carried out in DMF and were mediated by DCCI.

(b) Amino acid ester hydrochlorides were converted to the free esters by addition of a tertiary base, either triethylamine or N-methyl morpholine.

(c) HOBT was added at the coupling stage when fragment condensation involved a peptide having an optically active carboxy terminal amino acid.

(d) Couplings were allowed to proceed for 24 hours in the cold room at +4°C.

(e) After coupling, purification was effected by washing with acid and base to remove unchanged reactants.

(f) Alkaline saponifications were carried out in aqueous methanol with an autotitrator at pH 11.5 to 12.0 with *N*·NaOH.

(g) Benzyloxycarbonyl protecting groups were removed by hydrogenolysis in methanol/acetic acid with 10% palladium on charcoal.

(h) The resulting acetate salts from the above hydrogenolysis were converted to the corresponding hydrochlorides by an addition of methanolic hydrogen chloride.

(i) Benzyl protecting groups were removed by hydrogenolysis in methanol with 10% palladium on charcoal.

(j) Tertiary butyl and tertiary butyloxycarbonyl protecting groups were removed with N-hydrogen. chloride in acetic acid, in the presence of anisole to act as a scavenger. Cleavage was allowed to proceed for 60 to 90 minutes.

(k) The final peptides were isolated as their hydrochlorides and were lyophilised from aqueous solution.

Example 1

H.Tyr.*D*-Met.Gly.Phe(4-$NO_2$).Pro.NHEt

This was prepared according to the Scheme set out in Table 1. The product was first isolated as the hydrochloride addition salt and then purified on carboxymethylcellulose (CMC 52) by gradient elution with ammonium acetate buffers (0.001M to 5M). After lyophilisation from aqueous solution the hydrochloride addition salt had the following characterising data:

Rf: 0.45[1]; 0.60[3]; 0.57[4]

$[\alpha]_D^{25} = +3.1°$ (c = 0.2, in methanol)

The following peptides were prepared, with the characterising data respectively shown therefor, according to standard procedures in peptide chemistry analogous to those set out in the foregoing Example. C-Terminal derivatives are indicated according to convention, that is to say:

—NHEt : ethylamide

—OMe : methyl ester

TABLE 1

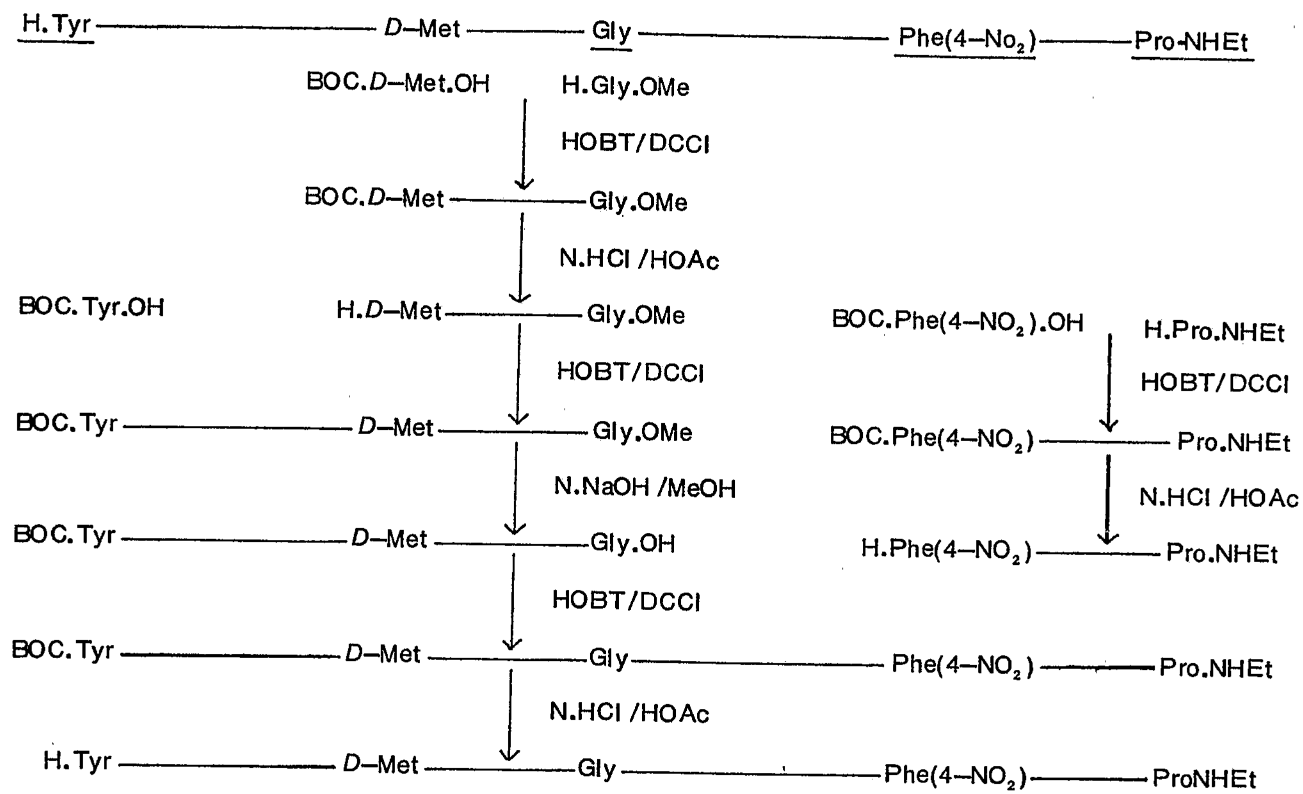
H.Tyr
D–Met
Gly
Phe(4–No$_2$)
Pro-NHEt
BOC.D–Met.OH
H.Gly.OMe
HOBT/DCCI
BOC.D–Met
Gly.OMe
N.HCl /HOAc
BOC.Tyr.OH
H.D–Met
Gly.OMe
BOC.Phe(4–NO$_2$).OH
H.Pro.NHEt
HOBT/DCCI
HOBT/DCCI
BOC.Tyr
D–Met
Gly.OMe
BOC.Phe(4–NO$_2$)
Pro.NHEt
N.NaOH /MeOH
N.HCl /HOAc
BOC.Tyr
D–Met
Gly.OH
H.Phe(4–NO$_2$)
Pro.NHEt
HOBT/DCCI
BOC.Tyr
D–Met
Gly
Phe(4–NO$_2$)
Pro.NHEt
N.HCl /HOAc
H.Tyr
D–Met
Gly
Phe(4–NO$_2$)
ProNHEt

| Ex. No. | Compound | Rf | $[\alpha]_D^{25}$ in methanol |
|---|---|---|---|
| 2 | H . Tyr . *D*-Met . Gly . Phe . *D*-Pro . NHEt . HCl | 0.78[3] | +59.0° (c = 0.2) |
| 3 | H . Tyr . *D*-Met . Gly . Phe(4-$NO_2$) . *D*-Leu . NHEt . HCl | 0.62[1]; 0.90[2] | +44.4° (c = 0.1) |
| 4 | H . Tyr . *D*-Met . Gly . Phe(4-$NO_2$) . *D*-Met . NHEt . HCl | 0.56[1]; 0.77[2] | +38.7° (c = 0.22) |

Example 5

Pharmacological Activity

Peptides of the foregoing Examples 1 to 4 were tested for the following activities according to standard pharmacological procedures.

(A) Analgesia in mice in the hot plate test (modification of the method of Eddy, N.B. et al., *J. Pharm. Exp. Therap.* (1953) *107*, 385, the peptide being administered by intracerebroventricular injection).

(B) Antidiarrhoeal activity in the rat. In this procedure rats were starved for 24 hours, the peptide then administered either subcutaneously or orally followed after 15 minutes by 1 ml castor oil per rat given orally.

(C) For antitussive testing, guinea-pigs are subjected to an aerosol containing 20% citric acid, — 30 minutes after administration of compound (orally or subcutaneously). The number of coughs during a five minute exposure are counted and meaned for six animals per treatment. The method is that described by Boura, A.L.A., Green, A.F. and Saunders, I.A. *Br. J. Pharmac.*, May 1970, Vol. 39, No. 1, page 225.

(D) Analgesia in mice in the writhing test (modification of the method of Hendershot L.C. and Forsaith J., *J. of Pharm. and Exp. Ther.* 1959, Vol. 125, page 237); the peptide being administered orally.

(E) Compounds are tested for anaesthetic effects following intracerebroventricular injection in mice. The animals are observed for loss of righting reflex (failure to roll over within 10 seconds of being turned on their backs), loss of pinnal reflex (failure to flick the ear in response to a light touch at the base of the pinna) and loss of corneal reflex (failure to blink when the cornea is touched lightly). Loss of all three reflexes constitutes anaesthesia. In addition, any other effects are noted. If a compound shows a degree of anaesthesia at the starting dose of 10 $\mu$g/mouse, further doses are done and an $ED_{50}$ calculated. Interesting compounds are then tested intravenously in the same manner.

From the data obtained the respective $ED_{50}$ figures were calculated (i.e. the dose required to elicit the appropriate effect in 50% of the animals).

TABLE 2

| Compound of Example No. | | RESULTS EXPRESSED AS $ED_{50}$ | | | | |
|---|---|---|---|---|---|---|
| | Mouse hot plate μg/mouse icv — ANALGESIA | Antidiarrhoea mg/kg (rat) s.c. | Antidiarrhoea mg/kg (rat) p.o. | Anti-tussive mg/kg (Guinea-pig) | Writhing mg/kg mouse — ANALGESIA | Anaesthesia μg/mouse icv |
| 1 | 0.007 | 0.1 | 1 | 7 p.o. | NT | None at 10 μg |
| 2 | 0.1 | 0.8 | None at 10 | NT | NT | None at 10 μg |
| 3 | 0.005 | 0.7 | None at 10 | NT | NT | >10 |
| 4 | 0.005 | 0.2 | 10 | NT | NT | None at 10 |

NT = not tested
None at . . . = no effect was observed at this dose

Example 6

Pharmaceutical Formulations

(A) *Tablet Formulation (20 mg/tablet)*

| | |
|---|---|
| Compound of formula (I) | 20 mg |
| Lactose | 76 mg |
| Maize Starch | 10 mg |
| Gelatin | 2 mg |
| Magnesium Stearate | 2 mg |

Mix together the compound of formula (I), Lactose and Maize Starch. Granulate with a solution of the Gelatin dissolved in water. Dry the granules, add the Magnesium Stearate and compress to produce tablets, 110 mg per tablet.

(B) *Suppository (5 mg/product)*

| | |
|---|---|
| Compound of formula (I) | 250 mg |
| Suppository Base (Massa Esterinum C) | to 100 g |

Melt the suppository base at 40°C.. Gradually incorporate the compound of formula (I) in fine powder form and mix until homogeneous. Pour into suitable moulds, 2 g per mould, and allow to set.

Massa Esterinum C is a commercially available suppository base consisting of a mixture of mono, di, and triglycerides of saturated vegetable fatty acids. It is marketed by Henkel International, Düsseldorf.

(C) *Pessary (5 mg/product)*

| | |
|---|---|
| Compound of formula (I) | 5 mg |
| Lactose | 400 mg |
| Providone | 5 mg |
| Magnesium Stearate | 5 mg |

Mix together the compound of formula (I) and Lactose. Granulate with a solution of Providone in 50% aqueous ethanol. Dry the granules add the Magnesium Stearate and compress on suitably shaped punches, 415 mg per pessary.

(D) *Freeze-dried Injection 100 mg/vial*

| | |
|---|---|
| Compound of formula (I) | 100 mg |
| Water for Injections | to 2.0 ml |

Dissolve the compound of formula (I) in the Water for Injections. Sterilise the solution by passage through a membrane filter, 0.2 $\mu$m pore size, collecting the filtrate in a sterile receiver. Fill into sterile glass vials, 2 ml/vial under aseptic conditions and freeze-dry. Close the vials with sterile rubber closures secured with an aluminium seal.

The injection is reconstituted prior to administration by the addition of a convenient volume of Water for Injections or sterile saline solution.

In the foregoing, the weight of the compound of formula (I) is in each instance calculated with reference to the peptide base.

**Claims**

1. A peptide of formula (I):

$$H.\ Tyr.\textit{D}\text{-}Met.\ Gly.X^4.X^5,NHR \qquad (I)$$

wherein

$X^4$ is *L*-phenylalanyl or *L*-4-nitrophenylalanyl;

$X^5$ is *D*-leucyl, *D*-methionyl, *D*-prolyl or *L*-prolyl; and

R is an alkyl group of 1 to 5 carbon atoms;

*provided that*

when $X^4$ is *L*-phenylalanyl

*then* $X^5$ is *D*-prolyl;

and acid addition salts thereof.

2. A compound according to claim 1 wherein $X^4$ is *L*-4-nitrophenylalnyl.

3. A compound according to either of claims 1 and 2 wherein $X^5$ is *L*-prolyl.

4. A compound according to any of claims 1 to 3 wherein R is methyl or ethyl.

5. H.Tyr.*D*-Met.Gly.Phe(4-$NO_2$).Pro.ethylamide or an acid addition salt thereof.

6. H.Tyr.*D*-Met.Gly.Phe.*D*-Pro.ethylamide or an acid addition salt thereof.

7. H.Tyr.*D*-Met.Gly.Phe(4-$NO_2$).*D*-Leu.ethylamide or an acid addition salt thereof.

8. H.Tyr.*D*-Met.Gly.Phe(4-$NO_2$).*D*-Met.ethylamide or an acid addition salt thereof.

9. A pharmacologically and pharmaceutically acceptable acid addition salt of a peptide according to any of claims 1 to 4.

10. The addition salt with hydrochloric acid or a peptide according to any of claims 5 to 8,

11. A pharmaceutical formulation comprising a peptide or a pharmacologically and pharmaceutically acceptable acid addition salt thereof, as defined in any of claims 1 to 10 together with an acceptable carrier therefor.

12. A formulation according to claim 11, suitable for administration by a route selected from oral, rectal, nasal, buccal, vaginal and parenteral.

13. A formulation according to claim 11 comprising the compound in solution in an aqueous medium.

14. A formulation according to any of claims 11 to 13 in unit dosage form.

15. A formulation according to claim 14 in the form of a tablet suitable for oral administration.

16. A formulation according to claim 14 in the form of a suppository suitable for rectal administration.

17. A formulation according to claim 14 in the form of a pessary suitable for vaginal administration.

18. A formulation according to claim 14 in the form of an aqueous sterile injection solution suitable for parenteral administration.

19. A freeze-dried formulation according to claim 18.

20. A formulation according to any of claims 14 to 19 wherein each unit dosage contains the compound in an amount in the range 0.125 $\mu$g to 2 g, calculated with reference to the peptide base.

21. A formulation according to any of claims 11 to 20 wherein the compound is as defined in any of claims 5 to 8.

22. A method for preparing a formulation as defined in any of claims 11 to 21 comprising admixture of the ingredients thereof and, if necessary, shaping of the product.

23. A peptide or an acid addition salt thereof as defined in any of claims 1 to 10 for use as a morphine-like agent.

24. A peptide of an acid addition salt thereof as defined in any of claims 1 to 10 for use in inducing analgesia.

25. A peptide or an acid addition salt thereof as defined in any of claims 1 to 10 for use in the treatment or prophylaxis of diarrhoea.

26. A peptide or an acid addition salt thereof as defined in any of claims 1 to 10 for use as an antitussive agent.

27. A peptide or an acid addition salt thereof as defined in any of claims 1 to 10 for use as an anaesthetic.

28. A peptide or an acid addition salt thereof as defined in any of claims 1 to 10 for use in inducing a state of neuroleptanalgesia.

29. A method for the preparations of a compound of formula (I) as defined in any of claims 1 to 10 comprising

(a) the reaction of a reagent (II)

$$H—Y^1—OH \qquad (II)$$

wherein $Y^1$ is selected from the radical Tyr and a partial radical sequence having the radical Tyr at its N-terminal end and from thereon corresponding to formula (I), with a reagent (III)

$$H—Y^2 \qquad (III)$$

wherein $Y^2$ corresponds to the balance of the above defined product compound, the reagents (II) and (III) being optionally protected and/or activated where and as appropriate; followed as appropriate by deprotection of the product; or

(b) the reaction of a corresponding peptide alkyl ester with an appropriate monoalkylamine; followed as appropriate by conversion of the product into the base or an acid addition salt thereof.

**Revendications**

1. Un peptide de formule (I):

H.Tyr.*D*-Met.Gly.$X^4$.$X^5$.NHR

dans laquelle

$X^4$ représente un radical *L*-phénylalanyle ou *L*-4-nitrophénylalanyle;

$X^5$ un radical *D*-leucyle, *D*-méthionyle, *D*-prolyle ou *L*-prolyle; et

R un alkyle en $C_1—C_5$;

avec la condition que

si $X^4$ est le radical *L*-phénylalanyle,

$X^5$ soit le radical *D*-prolyle;

ainsi que ses sels d'addition d'acides.

2. Composé selon la revendication 1 dans lequel $X^4$ est le radical *L*-4-nitrophénylalanyle.

3. Composé selon la revendication 1 ou 2 dans lequel $X^5$ est le radical *L*-prolyle.

4. Composé selon l'une quelconque des revendications 1 à 3 dans lequel R est un groupe méthyle ou éthyle.

5. Le H.Tyr.*D*-Met.Gly.Phe(4-$NO_2$).Proéthylamide ou un sel d'addition d'acide de ce peptide.

6. Le H.Tyr.*D*-Met.Gly.Phe.*D*-Pro.éthylamide ou un sel d'addition d'acide de ce peptide.

7. Le H.Tyr.*D*-Met.Gly.Phe(4-$NO_2$).*D*-Leu.éthylamide ou un sel d'addition d'acide de ce peptide.

8. Le H.Tyr.*D*-Met.Gly.Phe(4-$NO_2$).*D*-Met.éthylamide ou un sel d'addition d'acide de ce peptide.

9. Sel d'addition d'acide pharmacologiquement et pharmaceutiquement acceptable d'un peptide selon l'une quelconque des revendications 1 à 4.

10. Chlorhydrate d'un peptide selon l'une quelconque des revendication 5 à 8.

11. Formule pharmaceutique comprenant un peptide ou un sel d'addition d'acide de celui-ci pharmacologiquement et pharmaceutiquement acceptable, selon l'une quelconque des revendications 1 à 10, avec un véhicule acceptable.

12. Formule selon la revendication 11 pour administration par la voie orale, rectale, nasale, buccale, vaginale ou parentérale.

13. Formule selon la revendication 11 comprenant le composé en solution dans un milieu aqueux.

14. Formule selon l'une quelconque des revendications 11 à 13 sous forme d'unités de prise (doses unitaires).

15. Formule selon la revendication 14 sous la forme d'un comprimé pour administration orale.

16. Formule selon la revendication 14 sous la forme d'un suppositoire pour administration rectale.

17. Formule selon la revendication 14 sous la forme d'un pessaire pour administration vaginale.

18. Formule selon la revendication 14 sous la forme d'une solution aqueuse injectable stérile pour l'administration parentérale.

19. Formule lyophilisée selon la revendication 18.

20. Formule selon l'une quelconque des revendications 14 à 19 dans laquelle chaque dose unitaire contient le composé actif dans une proportion de 0,125 $\mu$g à 2 g, en peptide base.

21. Formule selon l'une quelconque des revendications 11 à 20 dans laquelle le composé actif est tel que défini à l'une quelconque des revendications 5 à 8.

22. Méthode de préparation d'une formule selon l'une quelconque des revendications 11 à 21, comprenant le mélange des divers ingrédients et, si nécessaire, la mise du mélange à la forme voulue.

23. Un peptide ou un sel d'addition de celui-ci selon l'une quelconque des revendications 1 à 10 comme agent ayant une action semblable à celle de la morphine.

24. Peptide ou sel d'addition d'acide de celui-ci selon l'une quelconque des revendications 1 à 10 comme agent analgésique.

25. Peptide ou sel d'addition d'acide de celui-ci selon l'une quelconque des revendications de 1 à 10 pour le traitement ou la prophylaxie de la diarrhée.

26. Peptide ou sel d'addition d'acide de celui-ci selon l'une quelconque des revendications de 1 à 10 comme agent anti-tussif.

27. Peptide ou sel d'addition d'acide de celui-ci selon l'une quelconque des revendications 1 à 10 comme agent anesthésique.

28. Peptide ou sel d'addition d'acide de celui-ci selon l'une quelconque des revendications de 1 à 10 pour induire un état de neuroleptanalgésie.

29. Procédé de préparation d'un composé de formule (I) selon l'une quelconque des revendications 1 à 10, comprenant:

(a) la réaction d'un réactif (II)

$$H—Y^1—OH \qquad (II)$$

$Y^1$ désignant le radical Tyr ou une séquence de radicaux partielle comprenant le radical Tyr à son extrémité N terminale, correspondant à la formule (I) avec un réactif (III)

$$H—Y^2 \qquad (III)$$

$Y^2$ représentant la partie restante du composé ci-dessus défini, les réactifs (II) et (III) pouvant être éventuellement protégés et/ou activés si cela est approprié; puis on déprotège éventuellement le produit formé, ou bien

(b) la réaction d'un ester alkylique du peptide correspondant avec une monoalkylamine appropriée, puis, selon le cas, on transforme le produit obtenu en base ou en un sel d'addition d'acide.

**Patentansprüche**

1. Peptid der Formel I

$$H.Tyr.D\text{-}Met.Gly.X^4.X^5.NHR \qquad (I)$$

worin

$X^4$ *L*-Phenylalanyl oder *L*-4-Nitrophenylalanyl ist,

$X^5$ *D*-Leucyl, *D*-Methionyl, *D*-Prolyl oder *L*-Prolyl ist und

R einen Alkylrest mit 1 bis 5 Kohlenstoffatomen bedeutet,
mit der Maßgabe, daß
sofern $X^4$ *L*-Phenylalanyl ist,
$X^5$ *D*-Prolyl ist,
und Säureadditionssalze davon.

2. Verbindung nach Anspruch 1, worin $X^4$ *L*-4-Nitrophenylalanyl ist.
3. Verbindung nach einem der Ansprüche 1 und 2, worin $X^5$ *L*-Prolyl ist.
4. Verbindung nach einem der Ansprüche 1 bis 3, worin R für Methyl oder Ethyl steht.
5. H.Tyr.*D*-Met.Gly.Phe(4-$NO_2$).Pro.Ethylamid oder ein Säureadditionssalz davon.
6. H.Tyr.*D*-Met.Gly.Phe.*D*-Pro.Ethylamid oder ein Säureadditionssalz davon.
7. H.Tyr.*D*-Met.Gly.Phe(4-$NO_2$).*D*-Leu.Ethylamid oder ein Säureadditionssalz davon.
8. H.Tyr.*D*-Met.Gly.Phe(4-$NO_2$).*D*-Met.Ethylamid oder ein Säureadditionssalz davon.
9. Pharmakologisch und pharmazeutisch verträgliches Säureadditionssalz eines Peptids nach einem der Ansprüche 1 bis 4.
10. Additionssalz mit Chlorwasserstoffsäure von einem Peptid nach einem der Ansprüche 5 bis 8.
11. Pharmazeutische Formulierung, enthaltend ein Peptid oder ein pharmakologisch und pharmazeutisch verträgliches Säureadditionssalz davon gemäß der Definition in einem der Ansprüche 1 bis 10, zusammen mit einem verträglichen Träger dafür.
12. Formulierung nach Anspruch 11, geeignet für die Verabfolgung auf oralem, rektalem, nasalem, buccalem, vaginalem oder parenteralem Weg.
13. Formulierung nach Anspruch 11, enthaltend die Verbindung in Lösung in einem wäßrigen Medium.
14. Formulierung nach einem der Ansprüche 11 bis 13 in Einheitsdosisform.
15. Formulierung nach Anspruch 14 in Form einer für die orale Verabfolgung geeigneten Tablette.
16. Formulierung nach Anspruch 14 in Form eines für die rektale Verabfolgung geeigneten Suppositoriums.
17. Formulierung nach Anspruch 14 in Form eines für die vaginale Verabfolgung geeigneten Pessars.
18. Formulierung nach Anspruch 14 in Form einer für die parenterale Verabfolgung geeigneten wäßrigen sterilen Injektionslösung.
19. Gefriergetrocknete Formulierung nach Anspruch 18.
20. Formulierung nach einem der Ansprüche 14 bis 19, worin jede Einheitsdosis die Verbindung in einer Menge im Bereich von 0,125 $\mu$g bis 2 g, berechnet unter Bezug auf die Peptidbase, enthält.
21. Formulierung nach einem der Ansprüche 11 bis 20, worin die Verbindung der Definition in einem der Ansprüche 5 bis 8 entspricht.
22. Verfahren zur Herstellung einer Formulierung gemäß der Definition in einem der Ansprüche 11 bis 21, umfassend ein Gemisch der Bestandteile davon, und gegebenenfalls Gestalten des Produkts.
23. Peptid oder Säureadditionssalz davon gemäß der Definition in einem der Ansprüche 1 bis 10 zur Verwendung als ein dem Morphin ähnliches Mittel.
24. Peptid oder Säureadditionssalz davon gemäß der Definition in einem der Ansprüche 1 bis 10 zur Verwendung bei der Induzierung von Analgesie.
25. Peptid oder Säureadditionssalz davon gemäß der Definition in einem der Ansprüche 1 bis 10 zur Verwendung bei der Behandlung oder Prophylaxe von Diarrhoe.
26. Peptid oder Säureadditionssalz davon gemäß der Definition in einem der Ansprüche 1 bis 10 zur Verwendung als ein Hustenmittel.
27. Peptid oder Säureadditionssalz davon gemäß der Definition in einem der Ansprüche 1 bis 10, zur Verwendung als ein Anästhetikum.
28. Peptid oder Säureadditionssalz davon gemäß der Definition in einem der Ansprüche 1 bis 10 zur Verwendung bei der Induzierung eines Zustands von Neuroleptanalgesie.
29. Verfahren zur Herstellung einer Verbindung der Formel I gemäß der Definition in einem der Ansprüche 1 bis 10, umfassend
(a) die Reaktion eines Reagens II

$$H—Y^1—OH \qquad (II)$$

worin $Y^1$ unter dem Rest Tyr und einer Teilrestsequenz mit dem Rest Tyr am N-endständigen Ende und von da aus entsprechend der Formel I, ausgewählt ist, mit einem Reagens III

$$H—Y^2 \qquad (III)$$

worin $Y^2$ dem Ausgleich der oben definierten Produktverbindung entspricht, wobei die Reagentien II und III wahlweise wo und wie es geeignet ist, geschützt und/oder aktiviert sind, woran sich, sofern es zweckmäßig ist, eine Deprotektion des Produktes anschließt, oder

(b) die Reaktion eines entsprechenden Peptidalkylesters mit einem geeigneten Monoalkylamin,

und gegebenenfalls anschließende Überführung des Produkts in die Base oder ein Säureadditionssalz davon.